# EUROPÄISCHE PATENTSCHRIFT

(11) **EP 1 325 764 B1**
(45) Veröffentlichungstag und Bekanntmachung des Hinweises auf die Patenterteilung: **22.11.2006**
(21) Anmeldenummer: 02090420.7
(22) Anmeldetag: 20.12.2002
(51) Int. Cl.: A61N 1/39, A61N 1/362, A61N 1/372

(54) **Atrialer Herzschrittmacher**
Atrial pacemaker
Stimulateur cardiaque auriculaire

(30) Priorität: 04.01.2002 US 345095 P
(43) Veröffentlichungstag der Anmeldung: 09.07.2003
(73) Patentinhaber: BIOTRONIK GmbH & Co. KG, 12359 Berlin (DE)
(72) Erfinder: Thong, Tran, Dr., Portland, Oregon 97229 (US)
(74) Vertreter: Lindner-Vogt, Karin L.

(56) Entgegenhaltungen:
- EP-A- 0 904 802
- US-A- 5 630 834
- US-A- 5 919 209

## Beschreibung

Die Erfindung betrifft einen Herzschrittmacher, der zur Abgabe von Stimulationsimpulsen an ein Atrium eines Herzens ausgebildet ist.

Ein solcher Herzschrittmacher umfasst üblicherweise einen Anschluss für wenigstens eine atriale Elektrodenleitung, mit der elektrische Impulse an ein Atrium eines Herzens übertragen und an das Herzgewebe (Myokard) im Atrium abgegeben werden können. Der Anschluss für die atriale Elektrodenleitung ist üblicherweise mit einem atrialen Stimulationsimpulsgenerator verbunden, der so ausgebildet ist, dass er zur Generierung atrialer Stimulationsimpulse angesteuert werden kann, die über die atriale Elektrodenleitung abzugeben sind. Zum Ansteuern des Stimulationsimpulsgenerators ist eine (atriale) Steuereinheit vorgesehen, die mit dem atrialen Stimula-tionsimpulsgenerator verbunden und ausgebildet ist, den Stimulationsimpulsgenerator zum Erzeugen und Abgeben von atrialen Stimulationsimpulsen anzusteuern. Im hier interessierenden Fall verfügt die Steuereinheit über einen AF-Präventationsmodus, in dem die atriale Steuereinheit den Stimulationsimpulsgenerator zum Erzeugen einer Stimulationsimpulsfolge ansteuert, die geeignet ist, einer atrialen Fibrillation (AF) vorzubeugen.

Der zuvor skizzierte Herzschrittmacher ist vorzugsweise ein ICD (implantable cardioverter/defibrillator) mit Overdrive-Stimulation zur präventiven Therapie von atrialer Fibrillation. Ein solcher ICD verfügt üblicherweise auch über einen Anschluss für eine ventrikuläre Elektrodenleitung und einen Stimulationsimpulsgenerator für ventrikuläre Stimulationsimpulse, die im Ventrikel eines Herzens wirksam werden.

Bei der vorliegenden Erfindung steht das Problem der atrialen Fibrillation (AF) im Vordergrund. Die atriale Fibrillation beeinträchtigt einen signifikanten Teil der älteren Bevölkerung. Man unterscheidet zwei Arten von atrialer Fibrillation, nämlich die vagalvermittelte atriale Fibrillation und die adrinergische atriale Fibrillation. Die erst genannte vagalvermittelte atriale Fibrillation tritt bei Patienten im entspannten Zustand auf, beispielsweise während der Verdauungszeiten, nach dem Essen oder während der Nacht oder in den frühen Morgenstunden. Vorläufige Ergebnisse klinischer Studien legen nahe, dass Schrittmacher oder die Fibrillatoren, die über einen Modus zum präventiven Stimulieren zur Kontrolle der atrialen Fibrillation verfügen, mittels sogenannten overdrivepacing in der Lage sind, zumindest die Dauer von Episoden vagalinduzierter atrialer Fibrillation zu verringern.

Die zur Beschränkung oder Vermeidung atrialer Fibrillation bekannte, atriale Overdrive-Stimulation beruht auf dem Gedanken, dauerhaft möglichst viele atriale Kontraktionen zu stimulieren und möglichst wenige natürliche (spontane intrinsische) Kontraktionen zuzulassen. Daher wird bei der Overdrive-Stimulation mit entweder einer variablen oder einer festen, erhöhten atrialen Rate stimuliert, beispielsweise 80 bis 90 Schlägen pro Minute. Im Falle der adaptiven, variablen Overdrive-Stimulation erfolgt die atriale Stimulation derart, dass das atriale Stimulationsintervall eine Länge von etwa 95 % eines jeweiligen natürlichen (intrinsischen) atrialen Intervalls entspricht. Ein Beispiel für einen solchen adaptiven Overdrive-Algorithmus ist als DDD+ Algorithmus der Anmelderin bekannt.

Ein Herzschrittmacher der alle Merkmale des Oberbegriffs des Anspruchs 1 aufweist, ist aus EP-A-0904802 bekannt.

Da das Erzeugen von Stimulationsimpulsen äußerst Energie-aufwendig ist, führt die Overdrive-Stimulation zu einem unerwünschten, hohen Stromverbrauch. Dies beeinträchtigt die Lebensdauer der Herzschrittmacher mit Overdrive-Stimulation.

Die vorliegende Erfindung hat zum Ziel, die Beeinträchtigung der Lebensdauer eines Schrittmachers im Zusammenhang mit der Minderung oder Verhinderung atrialer Fibrillationen zu verringern.

Zu diesem Zweck ist bei einem Herzschrittmacher der eingangs beschriebenen Art erfindungsgemäß ein Zeitgeber vorgesehen, der mit der atrialen Steuereinheit verbunden und ausgebildet ist, den AF-Präventationsmodus tageszeitabhängig ein- bzw. auszuschalten und dadurch das Erzeugen und Abgeben der atrialen Fibrillationen vorbeugenden Stimulationsimpulsfolge uhrzeitabhängig zu aktivieren bzw. zu deaktivieren.

Die Erfindung beruht auf der Erkenntnis, dass hier die eingangs genannten vagal-induzierten atrialen Fibrillationen tageszeitabhängig gehäuft auftreten, nämlich dann, wenn sich der Patient in einem Ruhezustand befindet, beispielsweise während der Verdauungszeit nach dem Essen oder in den frühen Morgenstunden.

Der Erfindung liegt weiterhin die Erkenntnis zugrunde, das atriale Fibrillationen den Patienten zwar beeinträchtigen, jedoch nicht unmittelbar tödlich sind.

In einer bevorzugten Ausführungsvariante besitzt der erfindungsgemäße Herzschrittmacher einen Uhrzeitspeicher für die Uhrzeit, in dem der AF-Präventationsmodus ein- bzw. ausgeschaltet ist.

Vorzugsweise ist der letztgenannte Herzschrittmacher ausgebildet, elektrische Signale vom Atrium eines Herzens aufzunehmen und weist hierzu einen atrialen Eingangsverstärker und einen mit diesem verbundenen Detektor für atriale Fibrillationen auf. Letztgenannter ist mit der Steuereinheit verbunden. Die Steuereinheit ist so ausgebildet, dass sie Tageszeitabschnitte des Auftretens atrialer Fibrillation ermittelt. Hierzu dienen ihr als Eingangssignale das Signal des Uhrzeitgebers und des atrialen Fibrilla-tionsdetektors. Als Ausgangssignal erzeugt die Steuereinheit Einschaltzeiten für den AF-Präventationsmodus und speichert diese in dem Uhrzeit-speicher. Die Steuereinheit ist hierbei so ausgebildet, dass die ermittelten und gespeicherten Umschaltzeiten den Tageszeiten mit gehäuftem Auftreten atrialer Fibrillationen entspricht.

Der Detektor für atriale Fibrillationen (AF-Detektor) kann mit einem p-Wellen-Detektor verbunden sein, welcher ausgebildet ist, mit einer atrialen Kontraktion einhergehende p-Wellen in einem intrakardialen Elektrokardiogramm (EKG) zu detektieren.

Die Steuereinheit ist vorzugsweise, zumindest zeitweise, mit einem Statistikspeicher verbunden und wirkt mit dem Statistikspeicher derart zusammen, dass Tageszeiten gehäuften Auftretens atrialer Fibrillationen für mehrere, unterschiedliche Tage gespeichert werden.

In eine besonderen Ausführungsvariante sind der Statistikspeicher und eine Auswerteeinheit zum Ermitteln der Ein- und Ausschaltzeiten Bestandteil eines externen Patientengerätes, welches eine Telemetrieeinheit aufweist, die einer entsprechenden Telemetrieeinheit im Implantat einer telemetrischen Verbindung zwischen der Auswerteeinheit des Patientengerätes und der Steuereinheit des Implantates ermöglicht.

Eine solche Anordnung mit externem, telemetrisch einbindbarem Patientengerät ermöglicht eine aufwendige Auswertung von Daten über das Auftreten atrialer Fibrillationen, ohne das Implantat, vor allem hinsichtlich des Energieverbrauchs, zu belasten.

In Bezug auf die letztgenannte Ausführungsvariante ist vorzugsweise ein Implantat vorgesehen, welches eine Telemetrieeinheit aufweist, die eine telemetrische Verbindung zwischen dem Implantat und einem externen Patientengerät, insbesondere zwischen der Steuereinheit des Implantats und der Auswerteeinheit des externen Patientengerätes, ermöglicht. Wie zuvor geschildert, ermöglicht eine derartige Anordnung, dass die Ein- und Ausschaltzeiten für den AF-Präventionsmodus durch die Auswerteeinheit im Patientengerät - auch mit Hilfe aufwendiger Algorithmen - ermittelt werden. Diese Ein- und Ausschaltzeiten werden dann vom Patientengerät über eine Telemetrieverbindung an das Implantat übermittelt und dort in dem Uhrzeitspeicher gespeichert.

Vorzugsweise ist der Uhrzeitspeicher zum Speichern eines n Bit langen Signals ausgebildet und wirkt derart mit der Steuereinheit zusammen, dass jedem Bit des Signals eine vorbestimmte Tageszeitdauer zugeordnet ist und der Zustand eines jeweiligen Signalbits (0 oder 1) vorgibt, ob der AF-Präventionsmodus für die dem jeweiligen Bit zugeordnete Zeit aus- oder eingeschaltet ist. Geeignet ist insbesondere ein 48-Bit-Speicher, bei dem jedes der 48 Bit für eine definierte Tageszeitdauer von halbstündiger Länge steht.

Bei einer derartigen, besonders einfachen Ausführungsform kann das Signal des Uhrzeitgebers ein einfaches Adresssignal für den 48-Bit-Speicher sein. Ausgelesen wird aus dem Speicher das jeweilige Bit (0 oder 1) und der atrialen Steuereinheit zugeführt. Wenn das ausgelesene Bit 1 ist, wird der AF-Präventionsmodus der atrialen Steuereinheit eingeschaltet, ist das ausgelesene Bit 0, bleibt der AF-Präventionsmodus ausgeschaltet.

Der Signalzustand des ausgelesenen Bits (0 oder 1) schaltet somit unmittelbar den AF-Präventionsmodus aus oder ein.

In einer Ausführungsform ist der Uhrzeitspeicher durch einen Arzt zu programmieren.

In einer alternativen Ausführungsvariante ist der Uhrzeitspeicher zum unmittelbaren Speichern von Ein- und Ausschaltzeitpunkten ausgebildet und wirkt derart mit der Steuereinheit zusammen, dass der AF-Präventionsmodus zu einer gespeicherten Einschaltzeit eingeschaltet wird und so lange eingeschaltet bleibt, bis der AF-Präventionsmodus zum nächst folgenden Ausschaltzeitpunkt wieder ausgeschaltet wird und bis zum nächsten Einschaltzeitpunkt ausgeschaltet bleibt. Bei dieser Ausführungsvariante ist somit nicht ein Bit eines im Uhrzeitspeicher gespeicherten Signals unmittelbar kennzeichnend für das Ein- oder Ausgeschaltetsein des AF-Präventionsmodus in der dem jeweiligen Bit zugeordneten Tageszeit. Vielmehr sind in dem Uhrzeitspeicher direkt Uhrzeiten als Ein- oder Ausschaltzeitpunkte (im Gegensatz zu den gewisse Zeiträume kennzeichnenden Bits) gespeichert. Diese Form der Zeitspeicherung ist zwar speicheraufwendiger, ermöglicht jedoch Ein- bzw. Ausschaltzeiten, die "krummen" Zeiträumen entsprechen und beispielsweise von einem Halbstunden-Raster abweichen. Auch diese letztgenannte Ausführungsvariante ist besonders geeignet für ein Implantat mit einer Telemetrieeinheit. Die Bestimmung der Ein- und Ausschaltzeitpunkte erfolgt dann analog der zuvor geschilderten Weise mittels einer Auswerteeinheit in einem, zumindest zeitweise, telemetrisch angebundenen externen Patientengerät.

Der Herzschrittmacher ist vorzugsweise ausgebildet, bei eingeschaltetem AF-Präventionsmodus atriale Stimulationsimpulsfolgen im Overdrive-Modus abzugeben.

Alternativ ist der Herzschrittmacher im AF-Präventionsmodus ausgebildet, Stimulationsimpulsfolgen mit einer erhöhten atrialen Rate von vorzugsweise 80 bis 90 Impulsen pro Minute auszulösen.

Die Erfindung soll nun anhand eines Ausführungsbeispiels mit Hilfe der Figuren näher beschrieben werden. Die Figuren zeigen:
- Figur 1: eine schematische Blockdarstellung der für die vorliegende Erfindung interessierenden Bestandteile eines Herzschrittmachers
- Figur 2: Bestandteile einer besonders einfachen Steuerung in schematischer Blockdarstellung
- Figur 3: ein Blockschaltbild eines implantierbaren Herzschrittmachers der in Figur 1 skizzierten Art mit zusätzlicher Telemetrieeinheit und entsprechendem, externen Patientengerät.

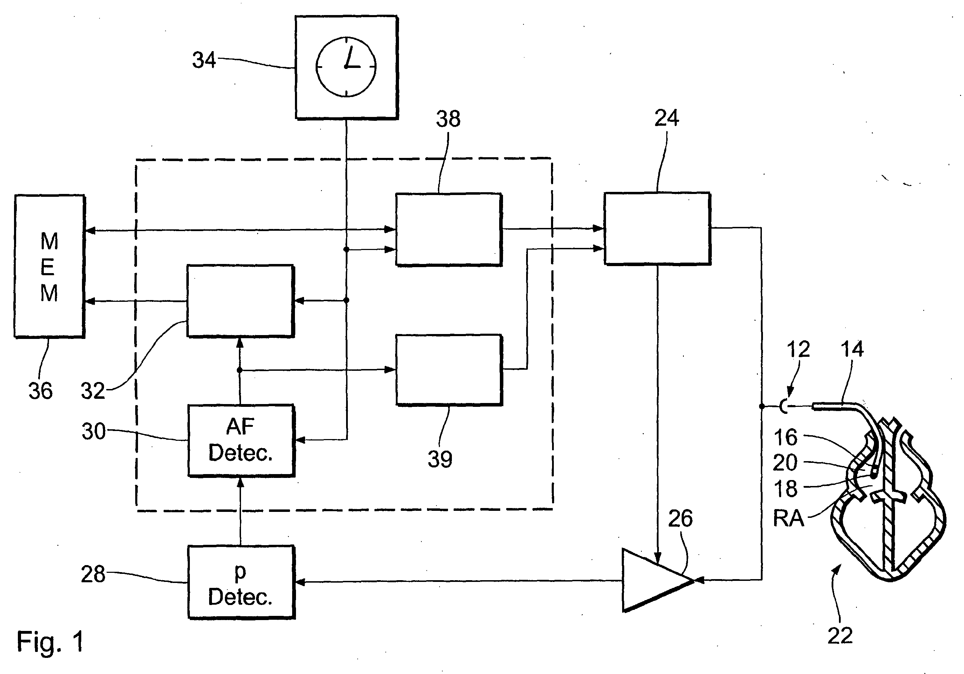

In Figur 1 sind in schematischer Darstellung lediglich die für die vorliegende Erfindung bedeutsamen Bestandteile eines Herzschrittmachers dargestellt. Dies sind ein Anschluss 12 für eine atriale Elektrodenleitung 14 mit im Ausführungsbeispiel zwei Elektroden 16 und 18. Die Elektrode 16 ist als Ringelektrode ausgebildet und die Elektrode 18 als Spitzelektrode. Das distale Ende der Elektrodenleitung 14 ist zur Anordnung im Atrium 20 eines Herzens 22 ausgebildet.

Die abgebildeten Bestandteile eines Herzschrittmachers umfassen einen Stimulationsimpulsgenerator 24 zum Erzeugen atrialer Stimulationsimpulse. Der Stimulationsimpulsgenerator 24 ist hierzu über den Anschluss 12 mit der Elektrodenleitung 14 verbunden.

Ebenfalls über den Anschluss 12 mit der Elektrodenleitung 14 ist ein Eingangsverstärker 26 verbunden. Der atriale Eingangsverstärker 26 ist dazu ausgebildet, im Atrium 20 eines Herzens 22 aufgenommene elektrische Signale zu verstärken und für die weitere Auswertung aufzubereiten.

Ausgangsseitig ist der Eingangsverstärker 26 mit einem p-Wellen-Detektor 28 verbunden, der dazu ausgebildet ist, für eine Kontraktion des Atriums 20 charakteristische p-Wellen in dem von dem Eingangsverstärker 26 aufbereiteten, intrakardialen Elektrokardiogramm zu detektieren.

Der p-Wellen-Detektor 28 ist ausgangsseitig mit einem AF-Detektor 30 für das Detektieren atrialer Fibrillationen verbunden. Der AF-Detektor 30 ermittelt hierzu die Frequenz des Auftretens von p-Wellen. Dies kann entweder durch Zählen der p-Wellen geschehen, die in einen durch einen Zeitgeber vorgegebenen definierten Zeitraum fallen. Alternativ kann mit jeder p-Welle ein Zeitgeber gestartet werden, der von der darauffolgenden p-Welle gestoppt wird, so dass auf diese Weise das Intervall zwischen zwei p-Wellen ermittelt wird. Unterschreitet dieses Intervall (beispielsweise 6 Zählschritte) einen vorgebenen Wert, entspricht dies einer erhöhten atrialen Rate und kann beispielsweise als atriale Fibrillation gewertet werden. Im Falle des Auftretens atrialer Fibrillationen gibt der AF-Detektor 30 ein ensprechendes Ausgangssignal aus, welches einer Speichersteuereinheit 32 zugeführt wird. Dieser Speichersteuereinheit 32 wird ebenfalls ein Ausgangssignal eines Uhrzeitgebers 34 zugeführt. Die Speichersteuereinheit 32 kann in unterschiedlichen Ausführungsvarianten verschieden ausgebildet sein.

In einer einfachen Ausführungsvariante ist die Speichersteuereinheit 32 ausgebildet, aus dem Uhrzeitsignal des Uhrzeitgebers 34 ein Adresssignal für einen Uhrzeitspeicher 36 zu generieren, falls seitens des AF-Detektors 30 ein eine atriale Fibrillation kennzeichnendes Signal anliegt. Die Speichersteuereinheit 32 veranlasst dann, dass unter der durch den Uhrzeitgeber determinierten Adresse ein Bit mit einem dem Einschalten des AF-Präventionsmodus entsprechenden Zustand, beispielsweise 1, gespeichert wird.

Bei dieser einfachen Ausführungsvariante ist eine atriale Stimulationssteuereinheit 38 ebenfalls mit dem Uhrzeitgeber 34 und dem Uhrzeitspeicher 36 verbunden und derart ausgebildet, dass sie den Uhrzeitspeicher 36 an der durch den Uhrzeitgeber 34 vorgegebenen Adresse ausliest und je nach Schaltzustand des ausgelesenen Bits (0 oder 1) einen AF-Präventionsmodus ein- oder ausschaltet. Ist beispielsweise das Bit unter der durch den Uhrzeitgeber 34 vorgegebenen Adresse des Uhrzeitspeichers 36 "1", nimmt die Stimulationssteuereinheit 38 den AF-Präventionsmodus an und steuert den Stimulationsimpulsgenerator 24 derart an, dass dieser Stimulationsimpulse mit einer erhöhten atrialen Rate, beispielsweise im Overdrive-Modus, generiert.

Ist das seitens der Stimulationssteuereinheit 38 entsprechend der Uhrzeit ausgelesene Bit aus dem Uhrzeitspeicher 36 "0", steuert die Stimulationssteuereinheit 38 den Stimulationsimpulsgenerator 24 nicht im AF-Präventionsmodus an. Je nach Ausbildung des Herzschrittmachers erfolgt die Ansteuerung des Stimulationsimpulsgenerators 24 durch die Stimulationssteuereinheit 38 in herkömmlicher Weise, d. h. es werden atriale Stimulationsimpulse generiert, wenn natürliche atriale Kontraktionen (und entsprechend durch den p-Wellen-Detektor 28 detektierte p-Wellen) innerhalb einer vorgegebenen Zeit, beispielsweise nach einem ventrikulären Ereignis, (VA-Zeit) nicht erfolgen. Da dies dem Stand der Technik entspricht, ist dies hier nicht weiter erläutert.

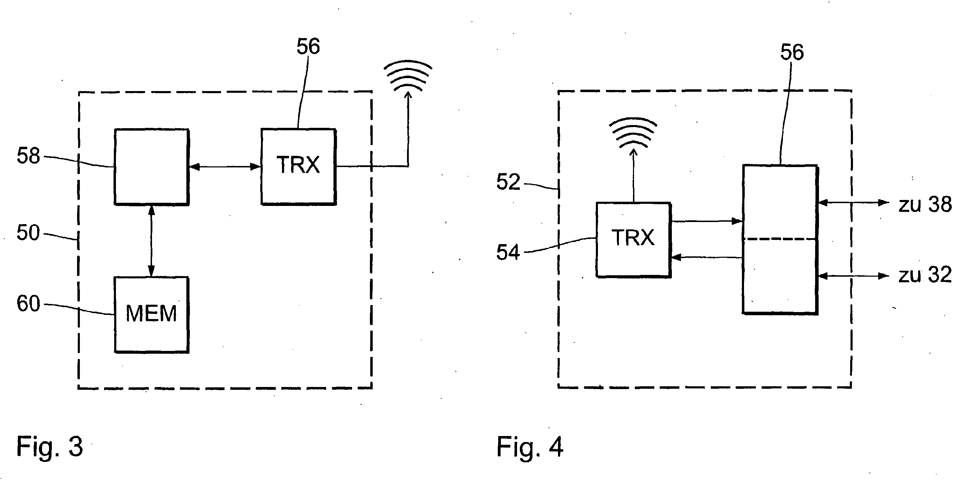

In einer im Zusammenhang mit Figur 3 weiter erläuterten Variante ist die Speichersteuereinheit 32 alternativ so ausgebildet, dass in dem Uhrzeitspeicher 36 die Uhrzeiten des Auftretens atrialer Fibrillationen zur weiteren Auswertung mittels einer Auswertereinheit in einem externen Patientengerät gespeichert werden.

Der Uhrzeitspeicher 36 ist bei der letztgenannten Ausführungsvariante zweiteilig ausgeführt. Neben der bereits geschilderten Speicherung von Zeiten des Auftretens atrialer Fibrillationen enthält der Uhrzeitspeicher 36 im zweiten Teil die Ein- und Ausschaltzeiten für den AF-Präventionsmodus. Bei dieser Ausführungsvariante ist die Stimulationssteuereinheit 38 so ausgebildet, dass der AF-Präventionsmodus aktiviert wird, wenn die durch den Uhrzeitgeber 34 gegebene Uhrzeit einer im zweiten Teil des Uhrzeitspeichers 36 gespeicherten Einschaltzeit entspricht. Umgekehrt wird der AF-Präventionsmodus wieder deaktiviert, wenn eine vom Uhrzeitgeber 34 vorgegebene Zeit einer im zweiten Teil des Uhrzeitspeichers 36 gespeicherten Ausschaltzeit entspricht. Im einfachsten Teil sind im zweiten Teil des Uhrzeitspeichers 36 Ein- und Ausschaltzeiten streng alternierend gespeichert, so dass mit jeder im Uhrzeitspeicher 36 gespeicherten Zeit ein Umschalten der Aktivierung des AF-Präventionsmodus von "Ein" zu "Aus" oder umgekehrt von "Aus" zu "Ein" erfolgt. Ein besonderes, eine Einschaltzeit von einer Ausschaltzeit unterscheidendes Bit ist dann nicht zu speichern.
Ebenso ist es möglich, für jeden Schaltzeitpunkt zusätzlich zu dem Uhrzeitsignal ein Bit zu speichern, welches den Schaltzeitpunkt als Einschaltzeitpunkt oder Ausschaltzeitpunkt modifiziert.

Nur der Vollständigkeit halber ist außerdem eine Defibrillationssteuereinheit 39 dargestellt, die auf den Fibrillationsdetektor 30 im Falle einer atrialen Fibrillation anspricht und den Stimulationsimpulsgenerator 24 zum Abgeben eines atrialen Defibrillationsimpulses ansteuern kann.

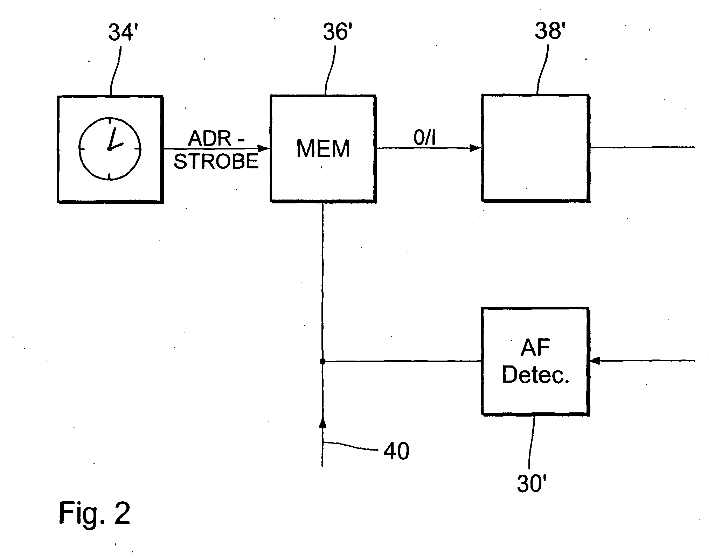

Figur 2 ist eine auszugsweise Darstellung einer besonders einfachen Ausführungsvariante, die der im Zusammenhang mit Figur 1 zuerst beschriebenen Ausführungsvariante ähnelt. Der Uhrzeitgeber 34' ist bei dieser Ausführungsvariante so ausgebildet, dass er unmittelbar einem Adresssignal für den Uhrzeitspeicher 36' generiert. Die Stimulationssteuereinheit 38' muss dann nur noch ein Read-Signal generieren, um den Uhrzeitspeicher 36' an der der aktuellen Uhrzeit entsprechenden Speicherstelle auszulesen. Wie zuvor beschrieben, wird beispielsweise nur ein einziges Bit ausgelesen, dessen Zustand, 0 oder 1, unmittelbar das Aus- oder Einschalten des AF-Präventionsmodus bewirkt.

Um eine Einschaltzeit in dem Uhrzeitspeicher 36' zu speichern, müssen ein Arzt über eine externe Schnittstelle 40 oder der AF-Detektor 30' nur ein Write-Signal generieren, um an der durch die jeweilige Uhrzeit vorgegebenen Speicherstelle das Bit auf den gewünschten Zustand zu setzen, beispielsweise "1" für das Einschalten des AF-Präventionsmodus.

In Figur 3 ist eine Ausführungsvariante dargestellt, bei der die Auswertung der Zeiten des Auftretens atrialer Fibrillationen und die Festlegung der Einund Ausschaltzeiten für den AF-Präventionsmodus mittels eines externen Patientengerätes 50 erfolgt. Das Patientengerät 50 ist über eine telemetrische Verbindung mit dem Herzschrittmacher 52 zu verbinden. Der Herzschrittmacher 52 entspricht im wesentlichen demjenigen, der bereits unter Figur 1 beschrieben worden ist. Der Uhrzeitspeicher 34" ist zweigeteilt. In einem Teil des Uhrzeitspeichers 36" (Figur 3 dem oberen Teil) sind die Ein- und Ausschaltzeiten für den AF-Präventionsmodus gespeichert. In dem anderen Teil des Uhrzeitspeichers 36" (in Figur 3 der untere Teil) sind die Zeiten des Auftretens atrialer Fibrillationen gespeichert. Der Uhrzeitspeicher 36" ist mit einem Telemetriesender und - empfänger 54 verbunden.

Das Patientengerät 50, was ebenfalls an den Telemetriesender 56 aufkomme, der zusammen mit dem implantatseitigen Telemetriesender 54 eine drahtlose Verbindung zwischen Herzschrittmacher 52 und Patientengerät 50 ermöglicht. Das Patientengerät 50 weist weiterhin eine Auswerteeinheit 58 auf, die mit einem Speicher 60 verbunden ist.

Die in Figur 3 abgebildete Anordnung ermöglicht es, im zweiten Teil des Uhrzeitspeichers 34" gespeicherte Zeiten des Auftretens atrialer Fibrillationen drahtlos an das Patientengerät 50 zu übermitteln. In dem Patientengerät 50 können diese Zeiten in dem Speicher 60 gespeichert werden und durch die Auswerteeinheit 58 zur Bildung von Ein- und Ausschaltzeiten für den AF-Präventionsmodus ausgewertet werden. Die so ermittelten Ein- und Ausschaltzeiten werden mit Hilfe der Telemetriesender und -empfänger 54 und 56 von der Auswerteeinheit 58 in den ersten Teil des Uhrzeitenspeichers 34" des Herzschrittmachers 52 übertragen. Diese Ein- und Ausschaltzeiten dienen dann wie bereits zu Figur 1 der zweiten Ausführungsvariante beschrieben, dem Ein- und Ausschalten des AF-Präventionsmodus.

## Patentansprüche

1. Herzschrittmacher, ausgebildet zur Abgabe von Stimulationsimpulsen an ein Atrium (20) eines Herzens (22), mit einem Anschluss (12) für eine atriale Elektrodenleitung (14) zur Abgabe elektrischer Impulse an das Atrium (20),
mit einem atrialen Stimulationsimpulsgenerator (24), der mit der Elektrodenleitung (14) verbindbar und ausgebildet ist, über die Elektrodenleitung (14) abzugebende atriale Stimulationsimpulse zu generieren,
mit einer Steuereinheit (38), die mit dem atrialen Stimulationsimpulsgenerator (24) verbunden ist und ausgebildet ist, den Stimulationsimpulsgenerator (24) zum Erzeugen und Abgeben von atrialen Stimulationsimpulsen anzusteuern, wobei die Steuereinheit (38) in einem AF-Präventionsmodus ausgebildet ist, den Stimulationsimpulsgenerator (24) zum Erzeugen einer Stimulationsimpulsfolge anzusteuern, die geeignet ist, einer atrialen Fibrillation (AF) vorzubeugen,
**gekennzeichnet durch** einen Zeitgeber (34),
der mit der Steuereinheit (38) verbunden und ausgebildet ist, den AF-Präventionsmodus tageszeitabhängig ein- bzw. auszuschalten und **dadurch** das Erzeugens und Abgeben der atrialen Fibrillationen vorbeugenden Stimulationsimpulsfolgen uhrzeitabhängig zu aktivieren bzw. zu deaktivieren.

2. Herzschrittmacher nach Anspruch 1, **gekennzeichnet durch** einen Uhrzeitspeicher (36) für Uhrzeiten des Ein- bzw. Ausschaltens des AF-Präventionsmodus.

3. Herzschrittmacher nach Anspruch 2, **dadurch gekennzeichnet, dass** der Uhrzeitspeicher (36) zum Speichern von n Bit ausgebildet ist und derart mit der Steuereinheit zusammenwirkt, dass jedes Bit einem bestimmten Zeitraum eines Tages zugeordnet und der Zustand eines jeweiligen Bit, nämlich 0 oder 1, den Aktivierungszustand des AF-Präventionsmodus, nämlich Ein oder Aus, für den entsprechenden Tageszeitraum bestimmt.

4. Herzschrittmacher nach Anspruch 3, **dadurch gekennzeichnet, dass** der Uhrzeitspeicher (36) zum Speichern von 48 Bit ausgebildet ist und derart mit der Steuereinheit (38) zusammenwirkt, dass jedes Bit einem jeweils anderen, halbstündigen Tageszeitraum zugeordnet ist.

5. Herzschrittmacher nach Anspruch 2, **dadurch gekennzeichnet, dass** der Uhrzeitspeicher (36) zum unmittelbaren Speichern von Ein- und Ausschaltzeitpunkten ausgebildet ist und derart mit der Steuereinheit (38) zusammenwirkt, dass der AF-Präventionmodus zu einer gespeicherten Einschaltzeit eingeschaltet wird und solange eingeschaltet bleibt, bis der AF-Präventionsmodus zum nächstfolgenden Ausschaltzeitpunkt wieder ausgeschaltet wird und bis zum nächsten Einschaltzeitpunkt ausgeschaltet bleibt.

6. Herzschrittmacher nach Anspruch 2, **gekennzeichnet durch** einen atrialen Eingangsverstärker (26) zur Aufnahme elektrischer Signale des Atriums (20) und einem mit diesem verbundenen Detektor (30) für atriale Fibrillationen, welcher mit der Steuereinheit (32) verbunden ist, wobei die Steuereinheit (32) ausgebildet ist, Tageszeitabschnitte des Auftretens atrialer Fibrillationen mittels der Signale des Uhrzeitgebers (34) und des atrialen Fibrillationsdetektors (30) zu ermitteln.

7. Herzschrittmacher nach Anspruch 6, **dadurch gekennzeichnet, dass** die Steuereinheit (38) dazu ausgebildet ist, aus den Tageszeitabschnitten des Auftretens atrialer Fibriliationen Einschaltzeiten für den AF-Präventionsmodus zu bilden und in dem Uhrzeitspeicher (36) zu speichern.

8. Herzschrittmacher nach einem der Ansprüche 2 bis 6, **dadurch gekennzeichnet, dass** der Herzschrittmacher wenigstens einen Telemetrieempfänger (54) aufweist, der zumindest mittelbar mit dem Uhrzeitspeicher (36) derart verbunden ist, dass Ein- und Ausschaltzeiten telemetrisch in den Uhrzeitspeicher (36) eingeschrieben werden können.

9. Herzschrittmacher nach Anspruch 6, **gekennzeichnet durch** einen Telemetriesender (56), der mit der Steuereinheit (32) und /oder dem Uhrzeitspeicher (36) in Verbindung steht und dazu ausgebildet ist, Tageszeitabschnitte des Auftretens atrialer Fibrillationen an ein externes Patientengerät (50) zu übermitteln.

10. Herzschrittmacher nach einem der Ansprüche 1 bis 9, **dadurch gekennzeichnet, das** die Steuereinheit (38) im AF-Präventionsmodus ausgebildet ist, das Erzeugen und Abgeben einer Stimulationsimpulsfolge mit einer erhöhten atrialen Rate von vorzugsweise 80 bis 90 Impulsen pro Minute auszulösen.

11. Herzschrittmacher nach einem der Ansprüche 1 bis 8, **dadurch gekennzeichnet, das** die Steuereinheit (38) im AF-Präventionsmodus ausgebildet ist, das Erzeugen und Abgeben einer Stimulationsimpulsfolge im Overdrive-Modus auszulösen.

12. System, umfassend einen Herzschrittmacher (52) nach Anspruch 8 und/oder 9, und ein externes Patientengerät (50), **dadurch gekennzeichnet, dass** das Patientengerät einen Telemetriesender und -empfänger (56;54) aufweist, der mit einer Auswerteeinheit (58) und einem Speicher (60) in Verbindung steht, wobei der Speicher (60) zum Speichern von Tageszeitabschnitten des Auftretens atrialer Fibrillationen ausgebildet und die Auswerteeinheit (58) dazu ausgebildet ist, aus den gespeicherten Tageszeitabschnitten des Auftretens atrialer Fibrillationen Ein- und Ausschaltzeiten für den AF-Präventionsmodus des Herzschrittmachers (52) zu bilden.

13. System nach Anspruch 12, **dadurch gekennzeichnet, dass** das Patientengerät (50) dazu ausgebildet ist, Ein- und Ausschaltzeiten für den AF-Präventionsmodus des Herzschrittmachers (52) telemetrisch an den Herzschrittmacher (52) zu übermitteln.

## Claims

1. A cardiac pacemaker, implemented for delivering stimulation pulses to an atrium (20) of a heart (22), having a terminal (12) for an atrial electrode line (14) for delivering electrical pulses to the atrium (20),
having an atrial stimulation pulse generator (24), which is connectable to the electrode line (14) and is implemented to generate atrial stimulation pulses to be delivered via the electrode line (14),
having a control unit (38), which is connected to the atrial stimulation pulse generator (24) and is implemented to activate the stimulation pulse generator (24) to generate and deliver atrial stimulation pulses, the control unit (38) being implemented in an AF prevention mode to activate the stimulation pulse generator (24) to generate a stimulation pulse sequence which is capable of avoiding an atrial fibrillation (AF),
**characterized by** a clock (34),
which is connected to the control unit (38) and is implemented to turn the AF prevention mode on and off as a function of the time of day and thus activate and deactivate the generation and delivery of the stimulation pulse sequences which avoid atrial fibrillations as a function of the time of day.

2. The cardiac pacemaker as recited in claim 1, **characterized by** a time of day memory (36) for times of day of turning the AF prevention mode on and off.

3. The cardiac pacemaker according to claim 2, **characterized in that** the time of day memory (36) is implemented for storing n bits and works together with the control unit in such way that each bit is assigned to a specific period of time of a day and the status of a particular bit, namely 0 or 1, determines the activation state of the AF prevention mode, namely on or off, for the corresponding time period of day.

4. The cardiac pacemaker according to claim 3, **characterized in that** the time of day memory (36) is implemented for storing 48 bits and works together with the control unit (38) in such way that each bit is assigned to a different, half-hour time period of day.

5. The cardiac pacemaker according to claim 2, **characterized in that** the time of day memory (36) is implemented for direct storage of turning-on and turning-off times and works together with the control unit (38) in such way that the AF prevention mode is turned on at a stored turning-on time and remains turned on until the AF prevention mode is turned off again at the next following turning-off time and remains turned off until the next turning-on time.

6. The cardiac pacemaker according to claim 2, **characterized by** an atrial input amplifier (26) for recording electrical signals of the atrium (20) and a detector (30), connected thereto, for atrial fibrillations, which is connected to the control unit (32), the control unit (32) being implemented to ascertain time of day sections of the occurrence of atrial fibrillations using the signals of the time of day clock (34) and the atrial fibrillation detector (30).

7. The cardiac pacemaker according to claim 6, **characterized in that** the control unit (38) is implemented for producing turning-on times for the AF prevention mode from the time of day sections of the occurrence of atrial fibrillations and storing them in the time of day memory (36).

8. The cardiac pacemaker according to one of claims 2 through 6, **characterized in that** the cardiac pacemaker has at least one telemetry receiver (54), which is at least indirectly connected to the time of day memory (36) in such way that turning-on and turning-off times may be written telemetrically into time of day memory (36).

9. The cardiac pacemaker according to claim 6, **characterized by** a telemetry transmitter (56), which is connected to the control unit (32) and/or the time of day memory (36) and is implemented for the purpose of transmitting time of day sections of the occurrence of atrial fibrillations to an external patient device (50).

10. The cardiac pacemaker according to one of claims 1 through 9, **characterized in that** the control unit (38) is implemented, in the AF prevention mode, to trigger the generation and delivery of a stimulation pulse sequence having an elevated atrial rate, preferably of 80 to 90 pulses per minute.

11. The cardiac pacemaker according to one of claims 1 through 8, **characterized in that** the control unit (38) is implemented, in the AF prevention mode, to trigger the generation and delivery of a stimulation pulse sequence in overdrive mode.

12. A system, comprising a cardiac pacemaker (52) according to claim 8 and/or 9, and an external patient device (50), **characterized in that** the patient device has a telemetry transmitter and receiver (56; 54), which is connected to an analysis unit (58) and a memory (60), the memory (60) being implemented to store time of day sections of the occurrence of atrial fibrillations and the analysis unit (58) being implemented for the purpose of producing turning-on and turning-off times for the AF prevention mode of the cardiac pacemaker (52) from the stored time of day sections of the occurrence of atrial fibrillations.

13. The system according to claim 12, **characterized in that** the patient device (50) is implemented for the purpose of telemetrically transmitting turning-on and turning-off times for the AF prevention mode of the cardiac pacemaker (52) to the cardiac pacemaker (52).

## Revendications

1. Pacemaker, conçu pour l'envoi d'impulsions de stimulation à un atrium (20) d'un coeur (22), avec un branchement (12) pour une ligne d'électrodes (14) atriale pour l'envoi d'impulsions électriques à l'atrium (20),
avec un générateur atrial d'impulsion de stimulation (24), qui peut être relié à la ligne d'électrodes (14) et est conçu pour générer des impulsions de stimulation atriales à envoyer par la ligne d'électrodes (14),
avec une unité de commande (38), qui est reliée au générateur atrial d'impulsion de stimulation (24) et est conçue pour activer le générateur d'impulsion de stimulation (24) destiné à générer et envoyer des impulsions de stimulation atriales, l'unité de commande (38) étant réalisée dans un mode de prévention AF, pour activer le générateur d'impulsions de stimulation (24) pour générer une séquence d'impulsion de stimulation, qui est appropriée pour prévenir une fibrillation atriale (AF),
**caractérisé par** un générateur de temps (34), qui est relié à l'unité de commande (38) et est conçu pour connecter et déconnecter le mode de prévention AF en fonction de l'heure du jour et pour activer et désactiver de ce fait la génération et l'envoi des séquences d'impulsions de stimulation prévenant les fibrillations atriales en fonction de l'heure.

2. Pacemaker selon la revendication 1, **caractérisé par** une mémoire d'heure (36) pour des heures de connexion ou de déconnexion du mode de prévention AF.

3. Pacemaker selon la revendication 2, **caractérisé en ce que** la mémoire d'heure (36) est conçue pour le stockage de n bits et coopère avec l'unité commande de telle sorte que chaque bit est attribué à une période définie d'une journée et l'état d'un bit concerné, c'est-à-dire 0 ou 1, détermine l'état d'activation du mode de prévention AF, c'est-à-dire marche ou arrêt, pour la période du jour correspondante.

4. Pacemaker selon la revendication 3, **caractérisé en ce que** la mémoire d'heure (36) est conçue pour le stockage de 48 bits et coopère avec l'unité de commande (38) de telle sorte que chaque bit est attribué à une autre période du jour respective d'une demi-heure.

5. Pacemaker selon la revendication 2, **caractérisé en ce que** la mémoire d'heure (36) est conçue pour le stockage direct de moments de connexion et de déconnexion et coopère avec l'unité de commande (38) de telle sorte que le mode de prévention AF est connecté pour un temps de connexion mémorisé et reste déconnecté jusqu'à ce que le mode de prévention soit à nouveau déconnecté pour le prochain moment de déconnexion et reste déconnecté jusqu'au prochain moment de connexion.

6. Pacemaker selon la revendication 2, **caractérisé par** un amplificateur d'entrée (26) atrial pour l'enregistrement de signaux électriques de l'atrium (20) et un détecteur (30) relié à celui-ci pour des fibrillations atriales, qui est relié à une unité de commande (32), l'unité de commande (32) étant réalisée pour déterminer des périodes de journée d'apparition de fibrillations atriales au moyen des signaux du générateur de temps (34) et du détecteur de fibrillation (30) atriale.

7. Pacemaker selon la revendication 6, **caractérisé en ce que** l'unité de commande (38) est conçue pour former à partir des périodes de journée d'apparition de fibrillations atriales des heures d'enclenchement pour le mode de prévention AF et de les stocker dans la mémoire d'heure (36).

8. Pacemaker selon l'une quelconque des revendications 2 à 6, **caractérisé en ce que** le pacemaker présente au moins un récepteur de télémétrie (54), qui est relié au moins indirectement à la mémoire d'heure (36) de telle sorte que les heures de connexion et de déconnexion peuvent être inscrites par télémétrie dans la mémoire d'heure (36).

9. Pacemaker selon la revendication 6, **caractérisé par** un émetteur de télémétrie (56), qui est en liaison avec l'unité de commande (32) et/ou la mémoire d'heure (36) et est conçu pour transmettre les périodes de jour d'apparition de fibrillations atriales à un appareil de patient (50) externe.

10. Pacemaker selon l'une quelconque des revendications 1 à 9, **caractérisé en ce que** l'unité de commande (38) est réalisé dans le mode de prévention AF pour déclencher la génération et l'envoi d'une séquence d'impulsions de stimulation avec une cadence atriale élevée de préférence de 80 à 90 impulsions par minute.

11. Pacemaker selon l'une quelconque des revendications 1 à 8, **caractérisé en ce que** l'unité de commande (38) dans le mode de prévention AF est réalisée pour déclencher la génération et l'envoi d'une séquence d'impulsions de stimulation dans le mode Overdrive.

12. Système comprenant un pacemaker (52) selon la revendication 8 et/ou 9, et un appareil de patient (50) externe, **caractérisé en ce que** l'appareil de patient présente un émetteur et récepteur de télémétrie (56, 54) qui est en liaison avec une unité d'analyse (58) et une mémoire (60), la mémoire (60) étant réalisée pour mémoriser des périodes de journée d'apparition de fibrillations atriales et l'unité d'analyse (58) est réalisée pour former à partir des périodes de journée mémorisées d'apparition de fibrillations atriales des heures de connexion et de déconnexion pour le mode de prévention AF du pacemaker (52).

13. Système selon la revendication 12, **caractérisé en ce que** l'appareil du patient (50) est réalisé pour transmettre les heures de connexion et de déconnexion pour le mode de prévention AF du pacemaker (52) par télémétrie au pacemaker (52).
